# EUROPEAN PATENT SPECIFICATION

(11) **EP 2 298 404 B1**
(45) Date of publication and mention of the grant of the patent: **10.08.2016**
(21) Application number: 10179388.3
(22) Date of filing: 20.06.2003
(51) Int. Cl.: A61M 5/32, A61M 25/06

(54) **Catheter and introducer needle assembly with needle shield**
Katheter und Einführnadelanordnung mit Nadelschutz
Cathéter et ensemble aiguille-introducteur doté d'une protection d'aiguille

(30) Priority: 20.06.2002 US 390499 P; 17.12.2002 US 320960
(43) Date of publication of application: 23.03.2011
(62) Divisional of application: 07117417.1
(73) Proprietor: Becton Dickinson and Company, Franklin Lakes, NJ 07417 (US)
(72) Inventor: Howell, Glade H., Sandy, UT 84094 (US); Harding, Weston F., Lehi, UT 84043 (US); Cindrich, Christopher N., Draper, UT 84020 (US); Sonderegger, Ralph, Farmington, UT 84025 (US); Frodsham, Joseph, Kaysville, UT 84037 (US)
(74) Representative: dompatent von Kreisler Selting Werner - Partnerschaft von Patent- und Rechtsanwälten mbB

(56) References cited:
- EP-A1- 0 554 841
- EP-A2- 0 750 915
- WO-A-01/93940
- US-A- 6 117 108

## Description

### BACKGROUND OF THE INVENTION

### 1. Field of the Invention

The subject invention relates to a needle shield assembly constructed to safely shield the sharp distal tip of a needle, and restrict distal movement of the needle tip via a tilting or "canting" plate after the tip is shielded.

### 2. Background of the Invention

Intravenous (IV) catheters are used for infusing fluid, such as normal saline solution, various medicaments and total parenteral nutrition, into a patient or withdrawing blood from a patient. Peripheral IV catheters tend to be relatively short, and are on the order of about one and one-half inches in length. A common type of IV catheter is an over the needle peripheral IV catheter. As its name implies, an over the needle catheter is mounted over an introducer needle having a sharp distal tip. The catheter and the introducer needle are assembled so that the distal tip of the introducer needle extends beyond the distal tip of the catheter with the bevel of the needle facing up away from the patient's skin.

The catheter and introducer needle assembly are inserted at a shallow angle through the patient's skin into a peripheral blood vessel (i.e., a smaller blood vessel that is not connected directly to the heart but is one of the branches of the central blood vessels that is directly connected to the heart). In order to verify proper placement of the assembly in the blood vessel, the clinician confirms that there is flashback of blood in the needle and in a flashback chamber located at the proximal end of the needle. Typically, the flashback chamber is formed as part of the needle hub. Once proper placement is confirmed, the clinician applies pressure to the blood vessel by pressing down on the patient's skin near the distal tip of the introducer needle and the catheter. This finger pressure occludes further blood flow through the introducer needle. The clinician withdraws the introducer needle, leaving the catheter in place, and attaches a fluid-handling device to the catheter hub. Once the introducer needle is withdrawn from the catheter, it is deemed a "blood contaminated sharp" and must be properly handled.

In recent years, there has been great concern over the contamination of clinicians with a patient's blood and a recognition that "blood contaminated sharps" must be immediately disposed. This concern has arisen, in part, to reduce the risks associated with spreading diseases that can be transmitted by the exchange of body fluids from an infected person to another person. Thus, it is desirable to avoid contact with the body fluid of an infected person. Various needle shields have been developed. Generally, such needle shields work for their intended purpose but could be improved. For example, some needle shields are bulky, difficult to use or require special features or techniques to be operative.

US 6,117,108 A discloses several embodiments of a needle and needle shield assembly. In a first embodiment the assembly comprises a needle shield in the form of a spring member that is contained in a catheter hub, or housing through which a needle extends. The function of the spring member is controlled by the needle. When the needle is retracted from the catheter it releases the force that had previously prevented movement of the needle shield within the catheter hub. This in term causes the needle guard to snap onto a position in which it is clamped onto the needle shaft and in which it's distal wall blocks access to the needle tip. In another embodiment the needle includes a static feature in form of an increased diameter bulge, which is sufficiently small to allow the needle to move axially along the catheter, but greater in diameter than an opening in the end wall of the needle guard so as to prevent any further axial movement of the needle out of the needle guard

WO 01/93940 A2 describes a catheter and introduce a needle assembly with a needle shield. The needle is provided with a static feature that cannot pass through an opening contained in a spring gate which is biased by a spring member radially with respect to the needle. In other embodiments the spring member consists of formed sheet material comprising a locking leg biased toward the needle. The locking leg allows proximal movement of the needle but it blocks distal movement so that after use the needle tip remains in a retracted position within a shield body.

EP 0 544 841 A1 describes a catheter introducer assembly including needle tip shield. The needle has a static feature of larger diameter than the remaining portions of the needle. A spring member is provided which has a transverse wall portion capable of covering the needle tip when the needle is in a retracted position. The needle is blocked between a said transversal wall portion and a distal wall portion of the spring member. The distal wall portion has a through hole which cannot be passed by the static feature of the needle.

It is an object of the invention to provide a needle and needle shield assembly having an integral washer, hinge and canting plate which commonly form an actuation arm.

### SUMMARY OF THE INVENTION

The needle and needle shield assembly of the present invention is defined by claim 1.

In accord with one aspect of the invention an over the needle catheter assembly includes a catheter adapter and a needle. The needle has a diameter and a distal tip, slidingly disposed within the catheter adapter. A needle shield assembly is slidably mounted on the needle. The needle shield assembly has an open distal end and an open proximal end through which the needle passes. A rigid plate, referred to as a "canting plate", is disposed within the needle shield assembly and has an unactivated first position and an activated second position. In the second position, the canting plate restricts needle movement. Means for retaining canting plate are provided. The canting plate retention means is in communication with the canting plate and responsive to proximal movement of the needle, whereby, when the needle tip is housed within the needle shield assembly, the canting plate retention means is actuated, causing distal movement of the needle to urge the canting plate from the unactivated first position to the activated second position.

In accord with certain implementations of this aspect of the invention, the canting plate retention means comprises a spring, a retention arm, and a retention washer. The spring may be selected from the group consisting of a coil spring, a wave washer, and a leaf spring or the like. The needle shield assembly may have a plurality of canting plates responsive to the canting plate retention means. The canting plate retention means may include a canting plate retention arm and a retention washer attached to the canting plate and having a built-in spring. The retention washer may be housed entirely within the shield. The canting plate retention means may include an elastomeric washer and an alignment arm. The elastomeric washer may have a truncated distal end. The catheter adapter and the shield may be held together by an interlock. A static feature may be provided on the needle. Wherein said interlock is released prior to or substantially simultaneous with the static feature on the needle contacting the shield proximal end. The length between the needle tip and the static feature is such that when said static feature contacts the shield proximal end, the needle tip is housed within the shield. The canting plate may contain a hole for passage of the needle and be located distally of the proximal end of the shield. The canting plate may be returned to an unactivated position when the needle is no longer urged in a distal direction.

Certain implementations of this aspect of the invention provide the canting member is a canting plate. The spring may be a leaf spring integrally formed with the canting member. The shield body, canting plate and spring may be integrally formed. The shield body may include a retention washer disposed at the proximal end of the shield body, and the retention washer defines an opening through which the needle passes.

The apparatus discussed above may be used with a needle including a feature having a diameter greater than the body of the needle. The opening in the retention washer is sized to permit the needle body to pass but to prevent the feature from passing therethrough. The shield body may include a retention arm that, when the needle shield assembly is within the housing, is biased radially inward to engage the canting member and, when the needle shield assembly is outside the housing, moves radially outward to disengage the canting member. The shield body may include a ledge, disposed opposite the retention arm, and abutting the canting member. The ledge may be integrally formed with the sidewall.

In accord with another aspect of the invention, a needle shield assembly includes a shield body having a sidewall, a proximal end and a distal end.

Certain implementations of this aspect of the invention provide that a retention washer is positioned at the proximal end of the housing and a hole having a selected hole size is disposed in the retention washer.

### BRIEF DESCRIPTION OF THE DRAWINGS

A preferred embodiment is illustrated in the drawings in which like reference numerals refer to like elements and in which:

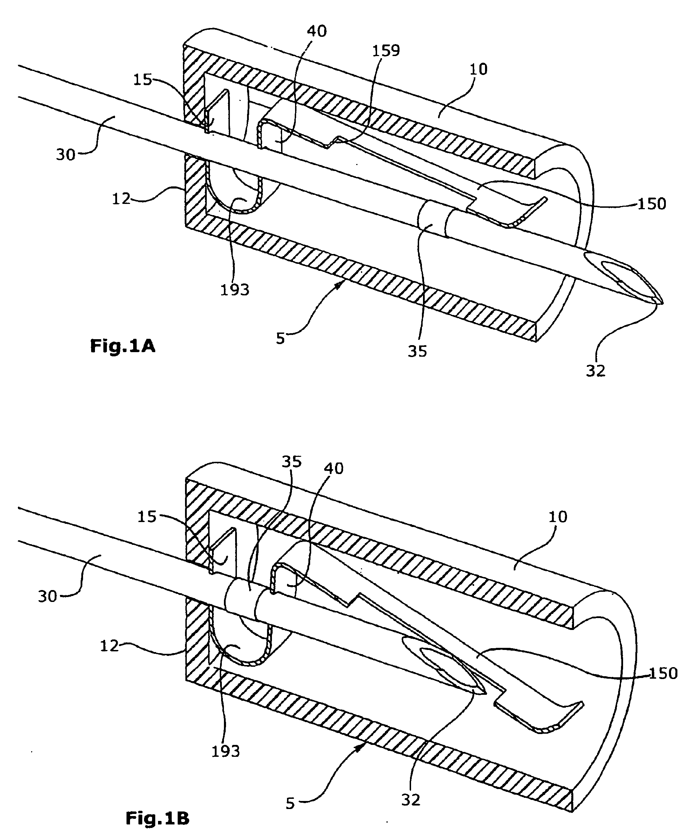

Fig. 1A is a front perspective view in partial cut-away of an embodiment of the invention in which the canting plate is integrally formed with a retention washer and a tip trigger, shown in an unactuated condition; and
Fig. 1B is a front perspective view of the embodiment shown in Fig. 1A in an actuated condition.

### DETAILED DESCRIPTION

As used herein, the term "proximal" refers to a location on the catheter and needle shield assembly of this invention closest to the clinician using the device and farthest from the patient in connection with whom the device is used when the device is used in its normal operation. Conversely, the term "distal" refers to a location on the catheter and needle shield assembly of this invention farthest from the clinician using the device and closest to the patient in connection with whom the device is used when the device is used in its normal operation.

### Integral Washer, Hinge and Canting Plate with Actuation Arm

Referring now to Figs. 1A and 1B an implementation of the invention is depicted including a retention washer 15 integrally formed with a flexural hinge 193 which in turn integrally formed with a canting plate 40 which is in turn integrally formed with an actuation arm 150. The retention washer is attached to the proximal end 12 of a shield body 10. In the unactuated condition, the canting plate is maintained in alignment with the needle 30 by the cooperation of the force exerted by the flexural hinge 193 and the restraint exerted by actuation arm 150. Specifically, the flexural hinge 193 acts as a spring urging the canting plate into a canted or engaging condition. This movement of the canting plate is prevented by the actuation arm which itself is engaged to the needle. See Fig. 20A. As the needle tip 32 is withdrawn, the actuation arm 150 comes out of engagement with the needle tip and is therefore free to move within the shield body 10. Consequently, the canting plate 40 succumbs to the bias exerted by the flexural hinge 193. As the canting plate is tilted out of alignment with the needle, it bindingly engages to the exterior of the needle. A cutout 159 may be provided on the actuation arm to permit movement of the actuation arm after passage of the needle tip without interference from the needle.

As disclosed above, certain implementations of the invention employ a feature 35 on the needle 30 to limit motion of the needle shield assembly 5 with respect to the tip 32 of the needle. Other implementations employ a tether to limit motion of the needle tip with respect to the needle shield assembly. It will be appreciated that in the various embodiments, the feature may be replaced with a tether (or the tether replaced with a feature) and still practice the invention. Further, the friction member is referred to, in certain implementations as an elastomeric washer. It will be appreciated that the friction member may be, or other materials having different properties and various shapes and still practice aspects of the invention.

The preceding description is exemplary rather than limiting in nature. Variations and modifications to the disclosed examples may become apparent to those skilled in the art that do not necessarily depart from the purview of this invention. For example, implementations of the invention may be employed with other needles, such as anesthesia needles or syringes or blood sample collection sets. The scope of legal protection given to this invention can only be determined by studying the following claims.

## Claims

1. A needle and needle shield assembly wherein said needle (30) has a tip (32), comprising:
a retention washer (15) integrally formed with a flexural hinge (193) which is in turn integrally formed with an actuating arm (150) selectively engaged to the needle,
wherein the retention washer (15), the flexural hinge (193) and the actuating arm (150) are positioned in a shield body (10) having a sidewall, a proximal end wall and a distal end, and
a feature (35) on the needle proximate to the tip (32) that is sized to prevent passage of the needle through an opening in the proximal end wall of the needle shield,
**characterized in that**
the flexural hinge (193) is integrally formed with a canting member (40) movable between an aligned condition and an off-alignment condition with the needle (30), wherein, in the aligned condition, the canting member (40) is maintained in alignment with the needle (30) by the cooperation of the force exerted by the flexural hinge (193) and the restraint exerted by the actuating arm (150), said canting member integrally formed with the actuating arm (150) selectively engaged to the needle (30), and being maintained by the actuation arm (150) in the aligned condition until the needle tip is proximally withdrawn, wherein, as the needle tip (32) is withdrawn, the actuating arm (150) comes out of engagement with the needle tip and is therefore free to move within the shield body (10), such that the canting member (40) succumbs to the bias exerted by the flexural hinge (193), such that it bindingly engages to the exterior of the needle.

## Patentansprüche

1. Nadel- und Nadelschutzanordnung, wobei die Nadel (30) eine Spitze (32) aufweist, mit:
einer Rückhalte-Unterlegscheibe (15), die einstückig mit einem Biegegelenk (193) ausgebildet ist, das seinerseits einstückig mit einem Betätigungsarm (150) ausgebildet ist, welcher wahlweise in Eingriff mit der Nadel ist,
wobei die Rückhalte-Unterlegscheibe (15), das Biegegelenk (193) und der Betätigungsarm (150) in einem Schutzkörper (10) mit einer Seitenwand, einer proximalen Stirnwand und einem distalen Ende angeordnet sind, und
einer an der Nadel nahe der Spitze (32) angeordneten Einrichtung (35), die derart bemessen ist, dass sie den Durchtritt der Nadel durch eine Öffnung in der proximalen Stirnwand des Nadelschutzes verhindert,
**dadurch gekennzeichnet, dass**
das Biegegelenk (193) einstückig mit einem Kippelement (40) ausgebildet ist, das zwischen einem mit der Nadel (30) fluchtenden Zustand und einem mit dieser nicht fluchtenden Zustand bewegbar ist, wobei im fluchtenden Zustand das Kippelement (40) durch das Zusammenwirken der von dem Biegegelenk (193) ausgeübten Kraft und der von dem Betätigungsarm (150) aufgebrachten Rückhaltekraft in Flucht mit der Nadel (30) gehalten ist, wobei das einstückig mit dem Betätigungsarm (150) ausgebildete Kippelement (40) selektiv in Eingriff mit der Nadel (30) ist, und durch den Betätigungsarm (150) im fluchtenden Zustand gehalten ist, bis die Nadelspitze in proximaler Richtung zurückgezogen wird, wobei beim Zurückziehen der Nadelspitze (32) der Betätigungsarm (150) außer Eingriff mit der Nadelspitze gelangt und daher frei in dem Schutzkörper (10) bewegbar ist, so dass das Kippelement (40) der von dem Biegegelenk (193) aufgebrachten Vorspannung derart nachgibt, dass es fest an der Außenseite der Nadel angreift.

## Revendications

1. Aiguille et ensemble protecteur d'aiguille où ladite aiguille (30) a une pointe (32), comprenant :
une rondelle de retenue (15) formée d'un seul tenant avec une charnière de flexion (193) qui est à son tour formée d'un seul tenant avec un bras d'actionnement (150) venant en prise de manière sélective avec l'aiguille,
où la rondelle de retenue (15), la charnière de flexion (193) et le bras d'actionnement (150) sont positionnés dans un corps de protection (10) ayant une paroi latérale, une paroi d'extrémité proximale et une extrémité distale, et
un élément (35) sur l'aiguille à proximité de la pointe (32) qui est dimensionné pour empêcher le passage de l'aiguille à travers une ouverture dans la paroi d'extrémité proximale du protecteur d'aiguille,
**caractérisé en ce que**
la charnière de flexion (193) est formée d'un seul tenant avec un organe inclinable (40) mobile entre un état aligné et un état hors alignement avec l'aiguille (30), où, dans l'état aligné, l'organe inclinable (40) est maintenu en alignement avec l'aiguille (30) par la coopération de la force exercée par la charnière de flexion (193) et la contrainte exercée par le bras d'actionnement (150), ledit organe inclinable étant formé d'un seul tenant avec le bras d'actionnement (150) venant en prise de manière sélective avec l'aiguille (30), et étant maintenu par le bras d'actionnement (150) dans l'état aligné jusqu'à ce que la pointe d'aiguille soit retirée de manière proximale, où, lorsque la pointe d'aiguille (32) est retirée, le bras d'actionnement (150) se détache de la pointe d'aiguille et est donc libre de se déplacer à l'intérieur du corps de protection (10), de sorte que l'organe inclinable (40) succombe à la sollicitation exercée par la charnière de flexion (193), de sorte qu'il vienne en prise de manière solidaire avec la partie extérieure de l'aiguille.
